# DEMANDE DE BREVET EUROPEEN

(11) **EP 1 195 389 A1**
(43) Date de publication de la demande: **10.04.2002**
(21) Numéro de dépôt: 01402383.2
(22) Date de dépôt: 17.09.2001
(51) Int. Cl.: C08F 20/34, C07C 213/08

(54) **Procédé pour augmenter la masse molaire d'un copolymère cationique de chlorure. d'Acryloyloxyethyltrimethylammonium, et copolymères correspondants.**

(30) Priorité: 05.10.2000 FR 0012730
(71) Demandeur: Atofina, 92800 Puteaux (FR)
(72) Inventeur: Zeh, Jean-Luc, 57350 Spicheren (FR); Sabatier, Lionel, 60270 Gouvieux (FR); Lepizzera, Stéphane, 57500 Saint-Avold (FR)
(74) Mandataire: Rieux, Michel

(57) **Abrégé**

Ce procédé pour augmenter la masse molaire d'un copolymère cationique du sel insaturé d'ammonium quaternaire de formule (I) : 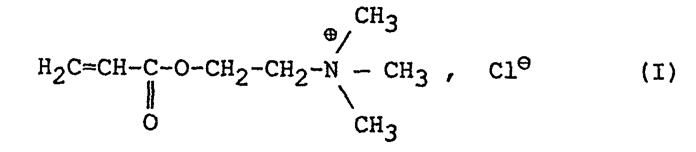 (ADAMQUAT MC),
est caractérisé par le fait que l'on utilise un monomère de formule (I) présentant une concentration en son dimère de formule (II) : 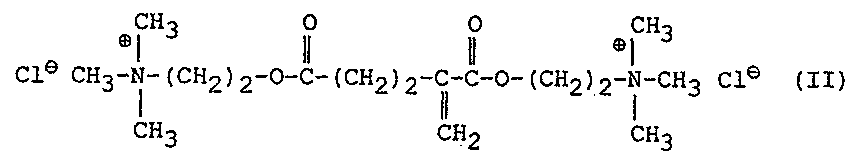 inférieure à 2000 ppm.

## Description

La présente invention porte sur un procédé pour augmenter la masse molaire d'un copolymère cationique de chlorure d'acryloyloxyéthyltriméthylammonium (ADAMQUAT MC). Elle porte également sur les copolymères cationiques correspondants ayant des masses molaires augmentées.

Les copolymères cationiques trouvent des applications industrielles dans différents domaines, tels que le traitement de l'eau, la fabrication du papier où ils sont utilisés comme agents de rétention, ... Dans toutes ces applications, l'utilisation d'un copolymère cationique de forte masse permet d'améliorer l'efficacité de celui-ci (amélioration de son rôle de floculant, meilleure rétention des fibres de papier) et de réduire son dosage.

La masse molaire des copolymères cationiques peut être contrôlée entre autres grâce au type de procédé utilisé, à la cinétique de polymérisation, à la nature et la pureté du comonomère ou encore à l'ajout d'agents réticulants. L'influence de ces paramètres sur la masse molaire des copolymères est maintenant connue et a fait l'objet de brevets. En revanche, même s'il est communément admis que la pureté de l'ADAMQUAT MC peut avoir une influence notable sur la masse molaire des copolymères cationiques, il n'existe pas actuellement de données précises permettant de relier la masse molaire des copolymères aux impuretés de l'ADAMQUAT MC.

La Société déposante a maintenant découvert de façon surprenante qu'il est possible d'obtenir des copolymères cationiques de fortes masses molaires grâce à un contrôle précis de la pureté de l'ADAMQUAT MC, en ayant montré que, parmi les différentes impuretés de l'ADAMQUAT MC, la présence du dimère de formule : 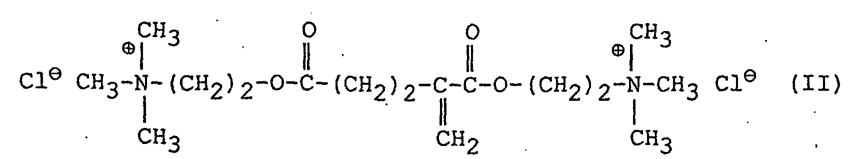 entraîne une baisse significative de la masse molaire des copolymères pour des concentrations supérieures à 1000-2000 ppm.

La présente invention a donc pour objet un procédé pour augmenter la masse molaire d'un copolymère cationique du sel insaturé d'ammonium quaternaire de formule (I) : 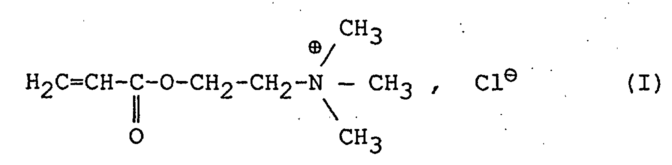 (ADAMQUAT MC),
caractérisé par le fait qu'on utilise un monomère de formule (I) présentant une concentration en son dimère de formule (II): 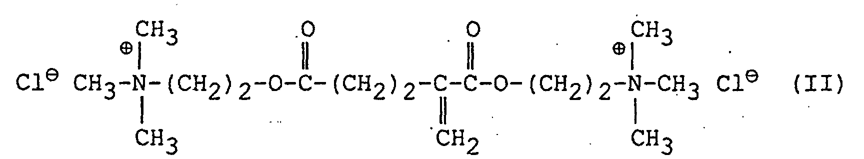 inférieure à 2000 ppm, en particulier inférieure à 1000 ppm.

L'utilisation d'un monomère (I) d'une telle pureté permet d'augmenter la viscosité d'une solution aqueuse des copolymères cationiques d'au moins 30% par rapport aux copolymères fabriqués avec un monomère (I) de pureté habituelle et dans les mêmes conditions de polymérisation (même procédé, mêmes comonomères, même proportion de comonomères).

La présente invention permet donc d'obtenir des copolymères cationiques de fortes masses molaires, en tout cas de masses molaires accrues par rapport aux copolymères fabriqués avec les monomères (I) de pureté habituelle.

Le monomère de formule (I) a notamment été préparé par réaction, en présence d'eau, de l'acrylate de N,N-diméthylaminoéthyle (ADAME) avec l'agent quaternisant CH₃Cl, ladite réaction ayant été conduite dans un réacteur fermé qui contenait la totalité de l'ADAME et qui avait été pressurisé par de l'air ou de l'air appauvri à 0,5 à 3 bars, en introduisant en continu, à la température de 35 à 65°C, d'une part, CH₃Cl, et, d'autre part, l'eau, jusqu'à l'obtention de la concentration souhaitée en le sel recherché dans l'eau, le démarrage de l'introduction de l'eau ayant commencé lorsque l'on a ajouté 0 - 20% de la quantité pondérale nécessaire à la réaction de CH₃Cl, et la pression en fin de réaction ayant pu atteindre 9 bars, après quoi l'on a dépressurisé le réacteur tout en maintenant une teneur en oxygène constante par introduction simultanée d'air, et après retour à la pression atmosphérique, on a éliminé le CH₃Cl résiduaire.

Conformément à d'autres caractéristiques particulières de ce procédé :
- on introduit l'agent quaternisant pendant un laps de temps de 1 - 7 heures et l'eau pendant un laps de temps de 2 - 8 heures ;
- on conduit la réaction avec un rapport molaire de l'agent quaternisant à l'ADAME de 1 à 1,1, de préférence de 1 à 1,05 ;
- on conduit la réaction avec un rapport moyen de débit eau/agent quaternisant de 0,1 - 1,2 en particulier de 0,3 - 0,8.

Par ailleurs, ce procédé peut être conduit en présence d'au moins un stabilisant, lequel peut être choisi parmi le 3,5-ditert.-butyl-4-hydroxytoluène, l'éther méthylique d'hydroquinone, l'hydroquinone, le catéchol, le tert.-butyl catéchol, la phénothiazine, et les mélanges de ces stabilisants, la teneur en agent(s) stabilisant(s) étant notamment de 20 à 2000 ppm, de préférence de 100 à 1200 ppm, par rapport à la solution aqueuse de sel quaternaire (I).

On peut ajouter en outre au milieu réactionnel au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

La présente invention a également pour objet un copolymère cationique obtenu à partir d'une composition de monomères comprenant un monomère de formule (I) : 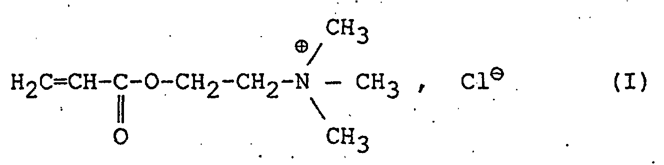 (ADAMQUAT MC)
présentant une concentration en son dimère de formule (II) : 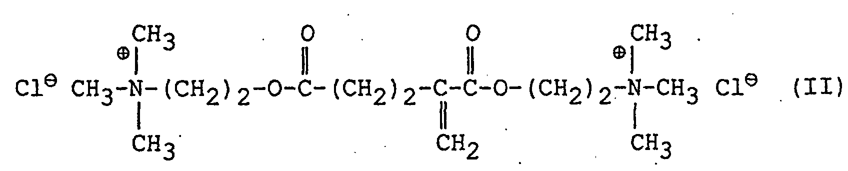 inférieure à 2000 ppm.

Le monomère (I) est notamment celui préparé par le procédé décrit ci-dessus.

Des copolymères cationiques selon la présente invention sont notamment ceux obtenus à partir d'une composition de monomères comprenant pour 100 parties en poids :
(A) jusqu'à 60 parties en poids, en particulier de 5 à 60 parties en poids, du monomère de formule (I) tel que défini ci-dessus ;
(B) 0 à 80 parties en poids d'au moins un monomère de formule (III) : 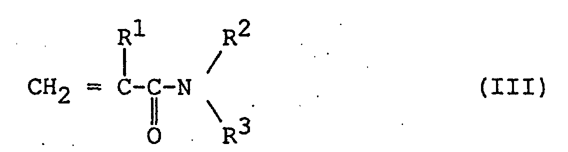 dans laquelle :
   - R¹ représente H ou -CH₃ ; et
   - R² et R³, identiques ou différents, représentent chacun H ou alkyle en C₁-C₅ ;
(C) 0 à 50 parties en poids d'au moins un monomère de formule (IV) : 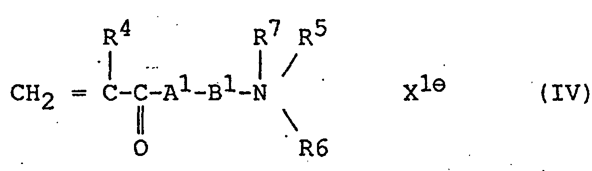 dans laquelle :
   - R⁴ représente H ou -CH₃ ; et
   - A¹ représente -O- ou -NH- ;
   - B¹ représente -CH₂-CH₂-, -CH₂CH₂CH₂-,
      CH₂-CHOH-CH₂- ;
   - R⁵ et R⁶ représentent chacun indépendamment -CH₃ ou-CH₂CH₃ ;
   - R⁷ représente H, -CH₃, -CH₂CH₃ ou -CH₂-C₆H₅ ;
   - X^{1⊖} représente un anion monovalent, tel que Cℓ^{⊖}, SCN^{⊖}, CH₃SO₃^{⊖} et Br^{⊖},
   à l'exclusion du composé de formule (I) ;
(D) 0 à 50 parties en poids d'au moins un monomère de formule (V) : 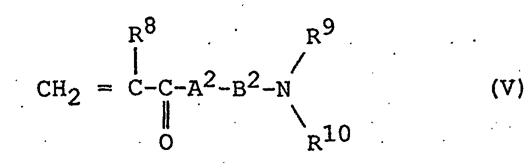 dans laquelle :
   - R⁸ représente H ou -CH₃ ;
   - A² représente -O- ou -NH- ;
   - B² représenté -CH₂-CH₂-, -CH₂CH₂CH₂-,
      -CH₂-CHOH-CH₂-;
   - R⁹ et R¹⁰ représentent chacun indépendamment -CH₃ ou-CH₂CH₃ ;
(E) 0 à 80 parties en poids d'au moins un monomère ayant une fonction carboxylique.

A titre d'exemples de monomères de formule (III), on peut citer l'acrylamide, le méthacrylamide, le N-méthylacrylamide et le N,N-diméthylacrylamide.

A titre d'exemples de monomères de formule (IV), on peut citer :
- le chlorure de méthacryloxyéthyldiméthyl benzyl ammonium (MADQUAT BZ) ;
- le chlorure de méthacryloxyéthyltriméthyl ammonium (MADQUAT MC) ; et
- le chlorure d'acryloxyéthyldiméthyl benzyl ammonium (ADAMQUAT BZ).

A titre d'exemples de monomères de formule (V), on peut citer le diméthylaminopropylacrylamide (DMAPAA) et le diméthylaminopropylméthacrylamide (DMAPMA).

A titre d'exemples de monomères (E), on peut citer l'acide acrylique, l'acide méthacrylique et l'acide itaconique.

La préparation des copolymères de la présente invention est une préparation classique, suivant le procédé en gel ou le procédé en émulsion inverse.
- le procédé en gel consiste à réaliser la polymérisation dans une solution aqueuse dans laquelle les monomères et polymères sont solubles et à aboutir en fin de polymérisation à un gel ; ce gel est ensuite séché et broyé pour obtenir une poudre prête à l'emploi ;
- le procédé en émulsion inverse consiste à réaliser la polymérisation en utilisant une phase dispersée aqueuse contenant les monomères et polymères hydrosolubles et une phase continue huileuse. Après polymérisation, l'émulsion inverse obtenue est inversée pour aboutir à une solution aqueuse et visqueuse contenant le polymère, prête à l'emploi.

Les Exemples suivants illustrent la présente invention sans toutefois en limiter la portée. Des ces exemples, les pourcentages sont en poids sauf indication contraire.

### EXEMPLE 1: SYNTHÈSE D'UN ADAMOUAT MC HAUTE PURETÉ

Dans un réacteur en verre de 1 l, à double enveloppe, spécialement conçu pour tenir la pression, équipé d'une sonde de température, d'un agitateur spécifique gaz-liquide (turbine avec axe creux), d'une soupape tarée à 10 bars, d'un disque d'explosion et de cannes plongeantes pour l'introduction des différents réactifs, on a chargé 429 g d'ADAME. Le réacteur a été fermé, puis pressurisé avec 1 bar d'air appauvri. L'agitation et le chauffage ont été mis en service.

Dès que la température a atteint 40°C (température de consigne : 47°C), on a commencé à introduire CH₃Cl à raison de 159 g/h. Dès que l'on a ajouté 15 g de CH₃Cl, soit 10% de la stoechiométrie en CH₃Cl, on a démarré l'introduction d'eau à un débit de 60 g/h tout en maintenant l'ajout de CH₃Cl. Le rapport de débit H₂O/CH₃Cl a été maintenu constant à 0,37 tout au long de la réaction.

Lorsque toute l'eau a été introduite (soit 1473 g), le réacteur a été ramené à la pression atmosphérique en utilisant le protocole suivant :
- dégazage du CH₃Cl en excès pendant 30 minutes avec introduction simultanée d'air dans la charge (débit : 3 Nl/h) ;
- retour progressif à la pression atmosphérique.

Les traces de CH₃Cl ont ensuite été éliminées par stripping à l'air (débit : 5Nl/h) pendant 30 minutes. Le réacteur a ensuite été mis en refroidissement, puis vidangé.

Les durées des différentes phases de la réaction étaient les suivantes :
- introduction de CH₃Cl : 1 h
- introduction H₂O : 2,3 h
- dégazage : 0,5 h
- stripping : 0,5 h
soit une durée totale d'environ 3,5 h.

Le brut réactionnel (716 g) a été analysé par chromatographie liquide haute performance (HPLC) pour déterminer la teneur en dimère (Formule II) de l'ADAMQUAT MC, qui était de 100 ppm.

### EXEMPLE 2: SYNTHÈSE D'UN ADAMOUAT MC DE PURETÉ CONVENTIONNELLE

On a procédé comme à l'Exemple 1 excepté que le CH₃Cl a été introduit en 7 h.

Le brut réactionnel (716 g) a été analysé par chromatographie liquide haute performance (HPLC) pour déterminer la teneur en dimère (Formule II) de l'ADAMQUAT MC, qui était de 3000 ppm.

### EXEMPLE 3: FABRICATION D'UN COPOLYMÈRE CATIONIOUE COMPARATIF (Procédé en gel)

L'ADAMQUAT MC 80 fabriqué selon l'Exemple 2 est polymérisé suivant le procédé en gel.

La polymérisation est réalisée de la façon suivante : 48 g d'acrylamide solide sont dissous dans 180 g d'eau. On ajoute alors 60 g de l'ADAMQUAT MC 80 à cette solution. Le mélange ainsi préparé est ensuite placé dans un récipient adiabatique du type Dewar. On ajoute enfin 0,005 g de persulfate d'ammonium et 0,005 g de métabisulfite de sodium pour faire démarrer la polymérisation à température ambiante. Le gel obtenu en fin de polymérisation est ensuite broyé et séché. La masse molaire moyenne en poids du copolymère est estimée en mesurant la viscosité d'une solution aqueuse molaire de NaCl contenant 0,1% de ce copolymère cationique avec un appareil Brookfield (DV-II, vitesse de rotation = 60 tpm, température = 20°C).

La viscosité de la solution molaire de NaCl contenant 0,1% du copolymère cationique fabriqué avec l'ADAMQUAT MC 80 de l'Exemple 2 est de 2,6 cps.

### EXEMPLE 4: FABRICATION D'UN COPOLYMÈRE CATIONIOUE SELON L'INVENTION (Procédé en gel)

La synthèse d'un copolymère cationique suivant le procédé décrit dans l'Exemple 3 avec l'ADAMQUAT MC 80 synthétisé selon l'Exemple 1 a été réalisée.

La viscosité de la solution molaire de NaCl contenant 0,1% du copolymère cationique fabriqué avec l'ADAMQUAT MC 80 de l'Exemple 1 est de 3,6 cps.

### EXEMPLE 5: FABRICATION D'UN COPOLYMÈRE CATIONIQUE COMPARATIF (Procédé en émulsion inverse)

L'ADAMQUAT MC 80 fabriqué selon l'Exemple 2 est polymérisé suivant le procédé de polymérisation en émulsion inverse.

La phase aqueuse est préparée en mélangeant 335,9 g d'acrylamide à 50%, 50 g de l'ADAMQUAT MC 80, 1,68 g de NaOH à 50%, 12,6 g de NaCl et 0,08 g d'acide éthylène diamine tétra acétique (EDTA) dans 176,35 g d'eau.

La phase huile est préparée en mélangeant 218,67 g d'hydrocarbures isoparaffiniques commercialisés par la Société "Exxon Chemical" sous la dénomination "Isopar M", 18,6 g de monooléate de sorbitan et 2,2 g de monooléate de sorbitan polyoxyéthyléné. La phase huile est chauffée à 40°C sous agitation pour faciliter la dissolution.

L'émulsion est alors préparée en mélangeant les phases huile et aqueuse à l'aide d'un mélangeur Ultra-Turrax® pendant 2 minutes à 10000 tpm.

L'émulsion est ensuite transférée dans un réacteur en verre à double enveloppe, muni d'un réfrigérant, d'une agitation centrale et d'une introduction d'azote. L'émulsion est chauffée à 46°C sous une agitation de 800 tpm et purgée à l'azote pendant 20 minutes. Une fois ce délai écoulé, 0,21 g d'azobisisobutyronitrile est introduit dans le réacteur. Une exothermie est alors observée et lorsque la température du milieu revient à 46°C, une cuisson de 1h à 54°C est réalisée, puis une seconde cuisson de 30 minutes à 57°C, et enfin une troisième cuisson de 2h à 78°C.

L'émulsion est ensuite refroidie et filtrée.

L'inversion de cette émulsion est effectuée de la façon suivante : 0,125 g de nonylphénol polyéthoxylé est ajouté à 490 g d'eau et mélangé avec un mélangeur Ultra-Turrax® . 10 g de l'émulsion sont injectés lentement dans cette solution aqueuse sous agitation avec le mélangeur Ultra-Turrax® . La solution inversée ainsi obtenue est laissée au repos pendant 24h.

La masse molaire moyenne du copolymère est estimée en mesurant la viscosité de la solution inversée avec un appareil Brookfield (ERV8, vitesse de rotation = 50 tpm, mobile 2, température = 20°C).

La viscosité de la solution aqueuse inversée du copolymère cationique fabriquée avec l'ADAMQUAT MC 80 de l'Exemple 2 est de 250 cps.

### EXEMPLE 6 : FABRICATION D'UN COPOLYMÈRE CATIONIQUE SELON L'INVENTION (Procédé en émulsion inverse)

La synthèse d'un copolymère cationique suivant le procédé décrit dans l'Exemple 5 avec l'ADAMQUAT MC 80 synthétisé selon l'Exemple 1 a été réalisée.

La viscosité de la solution aqueuse inversée du copolymère cationique fabriquée avec l'ADAMQUAT MC 80 de l'Exemple 1 est de 530 cps.

## Revendications

1. Procédé pour augmenter la masse molaire d'un copolymère cationique du sel insaturé d'ammonium quaternaire de formule (I) : 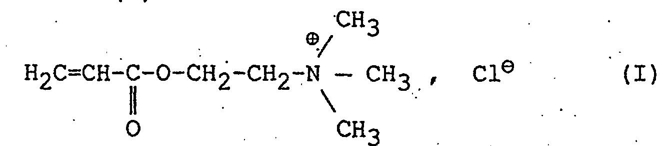 (ADAMQUAT MC),
**caractérisé par le fait que** l'on utilise un monomère de formule (I) présentant une concentration en son dimère de formule (II): 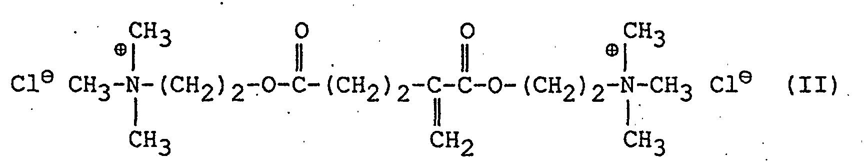 inférieure à 2000 ppm.

2. Procédé selon la revendication 1, **caractérisé par le fait que** l'on utilise un monomère de formule (I) présentant une concentration en son dimère de formule (II) inférieure à 1000 ppm.

3. Procédé selon l'une des revendications 1 et 2, **caractérisé par le fait que** l'on utilise un monomère de formule (I) qui a été préparé par réaction, en présence d'eau, de l'acrylate de N,N-diméthylaminoéthyle (ADAME) avec l'agent quaternisant CH₃Cl, ladite réaction ayant été conduite dans un réacteur fermé qui contenait la totalité de l'ADAME et qui avait été pressurisé par de l'air ou de l'air appauvri à 0,5 à 3 bars, en introduisant en continu, à la température de 35 à 65°C, d'une part, CH₃Cl, et, d'autre part, l'eau, jusqu'à l'obtention de la concentration souhaitée en le sel recherché dans l'eau, le démarrage de l'introduction de l'eau ayant commencé lorsque l'on a ajouté 0 - 20% de la quantité pondérale nécessaire à la réaction de CH₃Cl, et la pression en fin de réaction ayant pu atteindre 9 bars, après quoi l'on a dépressurisé le réacteur tout en maintenant une teneur en oxygène constante par introduction simultanée d'air, et après retour à la pression atmosphérique, on a éliminé le CH₃Cl résiduaire.

4. Copolymère cationique, **caractérisé par le fait qu'**il a été obtenu à partir d'une composition de monomères comprenant un monomère de formule (I) : 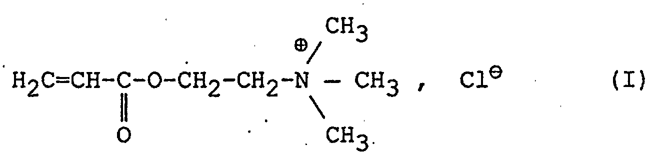 (ADAMQUAT MC)
présentant une concentration en son dimère de formule (II): 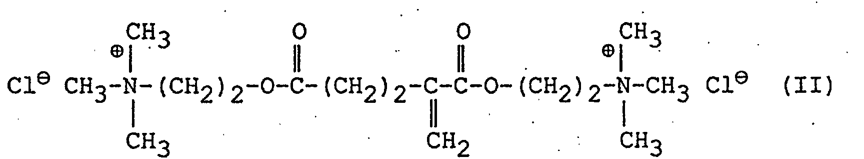 inférieure à 2000 ppm.

5. Copolymère cationique selon la revendication 4, **caractérisé par le fait que** le monomère de formule (I) présente une concentration en son dimère (II) inférieure à 1000 ppm.

6. Copolymère cationique selon l'une des revendications 4 et 5, **caractérisé par le fait que** le monomère de formule (I) a été préparé par réaction, en présence d'eau, de l'acrylate de N,N-diméthylaminoéthyle (ADAME) avec l'agent quaternisant CH₃Cl, ladite réaction ayant été conduite dans un réacteur fermé qui contenait la totalité de l'ADAME et qui avait été pressurisé par de l'air ou de l'air appauvri à 0,5 à 3 bars, en introduisant en continu, à la température de 35 à 65°C, d'une part, CH₃Cl et, d'autre part, l'eau, jusqu'à l'obtention de la concentration souhaitée en le sel recherché dans l'eau, le démarrage de l'introduction de l'eau ayant commencé lorsque l'on a ajouté 0 - 20% de la quantité pondérale nécessaire à la réaction de CH₃Cl, et la pression en fin de réaction ayant pu atteindre 9 bars, après quoi l'on a dépressurisé le réacteur tout en maintenant une teneur en oxygène constante par introduction simultanée d'air, et, après retour à la pression atmosphérique, on a éliminé le CH₃Cl résiduaire.

7. Copolymère cationique selon l'une des revendications 1 à 6, **caractérisé par le fait qu'**il a été obtenu à partir d'une composition de monomères comprenant pour 100 parties en poids :
(A) jusqu'à 60 parties en poids du monomère de formule (I) tel que défini à l'une des revendications 4 à 6 ;
(B) 0 à 80 parties en poids d'au moins un monomère de formule (III) : 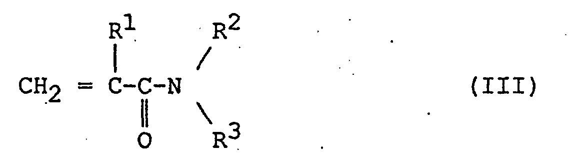 dans laquelle :
- R¹ représente H ou -CH₃ ; et
- R² et R³, identiques ou différents, représentent chacun H ou alkyle en C₁-C₅ ;
(C) 0 à 50 parties en poids d'au moins un monomère de formule (IV) : 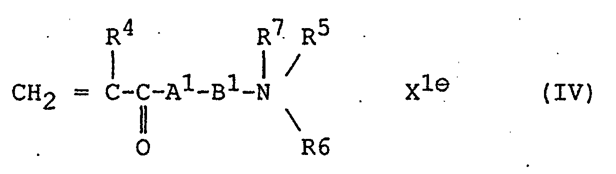 dans laquelle :
- R⁴ représente H ou -CH₃ ; et
- A¹ représente -O- ou -NH- ;
- B¹ représente -CH₂-CH₂-, -CH₂CH₂CH₂-,
CH₂-CHOH-CH₂- ;
- R⁵ et R⁶ représentent chacun indépendamment -CH₃ ou -CH₂CH₃ ;
- R⁷ représente H, -CH₃, -CH₂CH₃ ou -CH₂-C₆H₅ ;
- X^{1⊖} représente un anion monovalent, tel que Cℓ^{⊖}, SCN^{⊖}, CH₃SO₃^{⊖} et Br^{⊖},
à l'exclusion du composé de formule (I) ;
(D) 0 à 50 parties en poisd d'au moins un monomère de formule (V) : 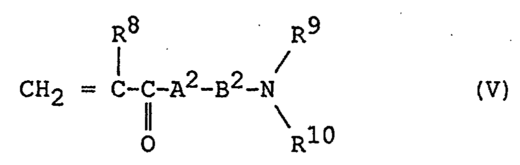 dans laquelle :
- R⁸ représente H ou -CH₃ ;
- A² représente -O- ou -NH- ;
- B² représente -CH₂-CH₂-, -CH₂CH₂CH₂-, -CH₂-CHOH-CH₂-;
- R⁹ et R¹⁰ représentent chacun indépendamment -CH₃ ou-CH₂CH₃ ;
(E) 0 à 80 parties en poids d'au moins un monomère ayant une fonction carboxylique.

8. Copolymère cationique selon la revendication 7, **caractérisé par le fait que** le monomère de formule (I) représente 5 à 60 parties en poids pour 100 parties en poids de la composition de monomères.
